(19) Europäisches Patentamt
European Patent Office
Office européen des brevets

(11) Veröffentlichungsnummer: **0 073 483**
**A2**

(12) # EUROPÄISCHE PATENTANMELDUNG

(21) Anmeldenummer: **82107848.2**

(22) Anmeldetag: **26.08.82**

(51) Int. Cl.³: **C 01 B 35/12**, C 01 B 33/28, B 01 J 29/28

(30) Priorität: **31.08.81 DE 3134316**

(43) Veröffentlichungstag der Anmeldung: **09.03.83**
**Patentblatt 83/10**

(84) Benannte Vertragsstaaten: **BE DE FR GB IT NL**

(71) Anmelder: **HOECHST AKTIENGESELLSCHAFT,**
**Postfach 80 03 20, D-6230 Frankfurt/Main 80 (DE)**

(72) Erfinder: **Baltes, Herbert, Dr., Johannesallee 24,**
**D-6230 Frankfurt am Main 80 (DE)**
Erfinder: **Leupold, Ernst Ingo, Dr., Am Zäunefeld 15,**
**D-6392 Neu-Anspach (DE)**
Erfinder: **Litterer, Heinz, Dr., Albert-Schweitzer-Allee 39,**
**D-6200 Wiesbaden (DE)**
Erfinder: **Wunder, Friedrich, Dr., Jahnstrasse 46,**
**D-6093 Flörsheim am Main (DE)**

(54) **Boro-Alumosilikate mit Zeolithstruktur und Verfahren zu deren Herstellung sowie ihre Verwendung.**

(57) Die Erfindung betrifft Boro-Alumosilikate mit Zeolith-Struktur und ein Verfahren zu deren Herstellung sowie ihre Verwendung. Zur Herstellung wird eine Mischung aus Silicium-, Aluminium-, Bor-, Natrium-, Kalium- und Tetramethylammoniumverbindungen sowie Wasser in spezifizierten Mengenverhältnissen hergestellt und in einem geschlossenen Gefäß erhitzt. Die Boro-Alumosilikate finden Verwendung als Katalysatoren bei der Herstellung von Kohlenwasserstoffen, insbesondere $C_2$- bis $C_4$-Olefinen, aus Methanol.

EP 0 073 483 A2

Boro-Alumosilikate mit Zeolithstruktur und Verfahren zu deren Herstellung sowie ihre Verwendung

Zeolithe sind kristalline Alumosilikate, bei denen durch eine dreidimensionale Verknüpfung von $SiO_4$- und $AlO_4$-Tetraedern regelmäßige Strukturen mit Hohlräumen und Poren entstehen. Im hydratisierten Zustand sind diese Poren und Hohlräume mit Wasser gefüllt. Dieses läßt sich ohne Beeinflussung der Kristallstruktur entfernen oder durch andere Moleküle ersetzen. Die negativen Ladungen der $AlO_4$-Tetraeder werden durch Kationen kompensiert. Diese können gegen andere positiv geladene Ionen ausgetauscht werden. Die geschilderten Eigenschaften ermöglichen die Verwendung der Zeolithe als Ionenaustauscher, Adsorbentien und Katalysatoren (D.W. Breck: Zeolite Molecular Sieves, 1974).

Zeolithe des X-, Y-, Mordenit-, Erionit- und Offretit-Typs beispielsweise besitzen als Katalysatoren für Umwandlungsreaktionen von Kohlenwasserstoffen wie Cracken, Hydrocracken oder Isomerisierungen beträchtliches technisches Interesse. Zeolithe vom Pentasil-Typ (z. B. Zeolith ZSM-5) gewinnen als Katalysatoren für die Umwandlung von Methanol zu Kohlenwasserstoffen steigende Bedeutung.

Aufgrund der zahlreichen Einsatzmöglichkeiten als Katalysatoren besteht großes Interessse an neuen Zeolithen mit spezifischen katalytischen Eigenschaften.

Beispielsweise erhält man sehr interessante Zeolithe, wenn man anstelle von Aluminium oder/und Silicium andere Elemente in das Zeolith-Gerüst einbaut. So wurden unter anderem Zeolithe der Pentasil-Reihe bekannt, die Bor (DE-OS 2 830 787), Eisen (DE-OS 2 831 611), Arsen (DE-AS 2 830 830), Antimon (DE-OS 2 830 787), Vanadin (DE-OS 2 831 631) oder Chrom (DE-OS 2 831 630) auf Tetraederplätzen enthalten.

0073483

Gegenstand der Erfindung sind Boro-Alumosilikate mit Zeolithstruktur, die dadurch gekennzeichnet sind, daß sie

a) die folgende Zusammensetzung besitzen:

$$SiO_2 : (0{,}20 \pm 0{,}10) \left[Al_2O_3 + B_2O_3\right] :$$

$$(0{,}15 \pm 0{,}1) \left[Na_2O + K_2O\right] : (0{,}15 \pm 0{,}1)\ R_2O$$

ausgedrückt in Molverhältnisssen von Oxiden, wobei R gleich Tetramethylammonium ist,

und

b) im Röntgenbeugungsdiagramm die in Tabelle 1 aufgeführten charakteristischen Signale aufweisen:

Tabelle 1

| Netzebenenabstände d [Å] | relative Intensität $I/I_o$ |
|---|---|
| 11,50 ± 0,2 | sehr stark |
| 7,56 ± 0,1 | mittel |
| 6,62 ± 0,1 | stark |
| 6,32 ± 0,1 | schwach |
| 5,74 ± 0,1 | schwach |
| 4,56 ± 0,1 | mittel |
| 4,33 ± 0,1 | stark |
| 3,76 ± 0,1 | sehr stark |
| 3,58 ± 0,1 | stark |
| 3,31 ± 0,1 | schwach |
| 3,15 ± 0,1 | mittel |
| 2,84 ± 0,1 | sehr stark |
| 2,68 ± 0,1 | schwach |
| 2,48 ± 0,1 | schwach |

Hierbei bedeutet $I_o$ die Intensität des stärksten Signals.

Für die Angabe der Intensitäten in Tabelle 1 gilt:

| relative Intensität | $100\ I/I_o$ |
|---|---|
| sehr stark | 80 - 100 |
| stark | 50 - 80 |
| mittel | 20 - 50 |
| schwach | 0 - 20 |

Das Aluminium-Bor-Verhältnis der erfindungsgemäßen Zeolithe beträgt im allgemeinen

$$\frac{Al_2O_3}{Al_2O_3 + B_2O_3} = 0,4 - 0,99 ,$$

vorzugsweise

$$\frac{Al_2O_3}{Al_2O_3 + B_2O_3} = 0,75 - 0,99 ,$$

ausgedrückt in Molverhältnissen der Oxide.

Die erfindungsgemäßen neuen Zeolithe besitzen eine dem Offretit (DE-OS 1 806 154) ähnliche Struktur, unterscheiden sich jedoch von diesem in der Zusammensetzung.

Die erfindungsgemäßen Zeolithe lassen sich herstellen, indem man Wasser und Silicium-, Aluminium-, Bor-, Natrium-, Kalium- und Tetramethylammoniumverbindungen mischt und in einem geschlossenen Gefäß erhitzt.

Die Ausgangsverbindungen werden im allgemeinen in folgendem Verhältnis eingesetzt, ausgedrückt in Molverhältnissen der Oxide:

$SiO_2$ : (0,04 ± 0,025) $Al_2O_3$ : (0,04 ± 0,025) $B_2O_3$ : (0,2 ± 0,08) $Na_2O$ : (0,2 ± 0,15) $K_2O$ : (0,1 ± 0,08) $R_2O$: (25 ± 10) $H_2O$

vorzugsweise im Verhältnis:

$SiO_2$ : (0,035 ± 0,01) $Al_2O_3$ : (0,05 ± 0,015) $B_2O_3$: (0,2 ± 0,08) $Na_2O$ : (0,2 ± 0,15) $K_2O$ : (0,1 ± 0,08) $R_2O$: (25 ± 10) $H_2O$

wobei R gleich Tetramethylammonium ist.

Als Verbindungen können beispielsweise eingesetzt werden: Kieselsäuregel, Kaliumsilicat, Natriumsilicat, Aluminiumhydroxid, Aluminiumsulfat, Borsäuretrioxid, Borsäure, Borax, Natriumhydroxid, Natriumsulfat, Kaliumhydroxid, Kaliumsulfat, Tetramethylammoniumhydroxid, Tetramethylammoniumchlorid. Aber auch andere Silicium-, Aluminium-, Bor-, Kalium-, Natrium- und Tetramethylammoniumverbindungen eignen sich für die Herstellung der erfindungsgemäßen Zeolithe.

Das Gemisch der jeweils gewählten Verbindungen mit Wasser wird im allgemeinen 12 bis 300 Stunden lang, vorzugsweise 24 bis 200 Stunden lang, auf eine Temperatur zwischen 60 und 150°C, insbesondere zwischen 80 und 140°C, in einem geschlossenen Gefäß erhitzt.

Die gebildeten kristallinen Zeolithe werden in üblicher Weise, z. B. durch Filtration, isoliert, gewaschen und getrocknet. Sie können nach bekannten Methoden in die katalytisch aktiven Formen überführt werden, z. B. durch Kalzinierung und/oder Ionenaustausch (D.W. Breck, Zeolite Molecular Sieves, 1974).

Die erfindungsgemäßen Zeolithe zeigen nach ihrer Überführung in die katalytisch aktive Form insbesondere bei der Umwandlung von Methanol zu Kohlenwasserstoffen, insbesondere $C_2$ bis $C_4$-Olefinen, eine wesentlich geringere Koksabscheidung und deutlich höhere Aktivität als die borfreien Offretite gemäß DE-OS 1 806 154. Es ist überraschend, daß man mit Hilfe der angegebenen Methode überhaupt Zeolithe mit den erfindungsgemäßen Merkmalen erhält.

Die Erfindung soll durch die folgenden Beispiele erläutert werden, wobei die Beispiele aber in keiner Weise einschränkend sein sollen. Alle angegebenen Röntgenbeugungsdaten wurden mit einem computergesteuerten Pulverdiffraktometer D-500 der Firma Siemens aufgenommen. Es wurde Kupfer-K-$\alpha$-Strahlung verwandt.

Beispiel 1

In eine Lösung aus 1,6 g Bortrioxid, 7,2 g Natriumhydroxid, 17,8 g Kaliumhydroxid, 4,5 g Natriumaluminat (54 Gew.% $Al_2O_3$, 41 Gew.% $Na_2O$) und 4,2 g Tetramethylammoniumchlorid in 125 g Wasser werden 98 g 40 Gew.%iges kolloidales Kieselgel eingebracht. Die entstandene Suspension wird durch intensives Rühren homogenisiert und 95 h lang auf 95°C in einem geschlossenen Gefäß erhitzt. Das erhaltene Produkt wird abfiltriert, mit Wasser gewaschen und bei 120°C getrocknet.

Die chemische Analyse ergibt folgende Zusammensetzung, ausgedrückt in Molverhältnissen von Oxiden:

$SiO_2$ : 0,112 $Al_2O_3$ : 0,012 $B_2O_3$ : 0,050 $Na_2O$ : 0,120 $K_2O$ : 0,041 $R_2O$

wobei R= Tetramethylammonium ist.

Das Ergebnis der Röntgenbeugungsanalyse ist in Tabelle 2 wiedergegeben.

Tabelle 2

| Netzebenenabstände d [Å] | relative Intensität $I/I_o$ |
|---|---|
| 11,50 | 88 |
| 7,54 | 20 |
| 6,60 | 74 |
| 6,32 | 14 |
| 5,73 | 6 |
| 4,56 | 29 |
| 4,32 | 53 |
| 3,75 | 100 |
| 3,58 | 68 |
| 3,30 | 17 |
| 3,15 | 36 |
| 2,84 | 87 |
| 2,67 | 18 |
| 2,48 | 11 |

Beispiel 2

2,3 g Borax, 8,5 g Natriumhydroxid, 17,8 g Kaliumhydroxid und 2,3 g Natriumaluminat (54 Gew.% $Al_2O_3$, 41 Gew.% $Na_2O$) werden in 125 ml Wasser gelöst. Zu dieser Lösung gibt man unter Rühren 98 g 40 Gew.-%iges kolloidales Kieselgel. Diese Suspension wiederum wird mit 14,0 g 25 Gew.-%iger wäßriger Tetramethylammoniumhydroxid-Lösung versetzt. Das entstandene Gemisch wird nach dem Homogenisieren 72 h in einem geschlossenen Gefäß auf 105°C erhitzt. Nach Aufarbeitung wie in Beispiel 1 erhält man ein kristallines Produkt der folgenden Zusammensetzung, ausgedrückt in Molverhältnissen von Oxiden:

$SiO_2 : 0,107\ Al_2O_3 : 0,016\ B_2O_3 : 0,048\ Na_2O : 0,123\ K_2O : 0,046\ R_2O$
wobei R = Tetramethylammonium ist.

Die Röntgendaten entsprechen den in Tabelle 1 angegebenen.

Beispiel 3

Es wird eine Suspension aus 3,2 g Bortrioxid, 21,4 g Natriumhydroxid, 35,6 g Kaliumhydroxid, 1,4 g Aluminiumhydroxid, 8,4 g Tetramethylammoniumchlorid, 250 g Wasser und 195 g 40 Gew.-%igem kolloidalem Kieselgel hergestellt und 190 h lang in einem geschlossenen Gefäß auf 95°C erhitzt. Nach Aufarbeitung wie in Beispiel 1 erhält man ein kristallines Produkt mit folgender Zusammensetzung, ausgedrückt in Molverhältnissen von Oxiden:

$SiO_2 : 0,100\ Al_2O_3 : 0,034\ B_2O_3 : 0,053\ Na_2O : 0,118\ K_2O : 0,045\ R_2O$

wobei R= Tetramethylammonium ist.

Die Röntgendaten entsprechen den in Tabelle 1 angegebenen.

Beispiel 4

Es wird eine Suspension aus 4,8 g Bortrioxid, 21,4 g Natriumhydroxid, 35,6 g Kaliumhydroxid, 0,7 g Aluminiumhydroxid, 8,4 g Tetramethylammoniumchlorid, 250 g Wasser und 195 g 40 Gew.%igem kolloidalem Kieselgel hergestellt und 570 h lang in einem geschlossenen Gefäß auf 95°C erhitzt. Nach Aufarbeitung wie in Beispiel 1 erhält man ein kristallines Produkt mit folgender Zusammensetzung, ausgedrückt in Molverhältnissen von Oxiden:

$SiO_2 : 0,058\ Al_2O_3 : 0,060\ B_2O_3 : 0,051\ Na_2O : 0,118\ K_2O : 0,047\ R_2O$
wobei R= Tetramethylammonium ist.

Das Produkt besitzt eine Offretit-Struktur (Röntgenspektrum wie in Tabelle 1).

Patentansprüche HOE 81/F 221

1. Boro-Alumosilicate mit Zeolithstruktur, dadurch gekennzeichnet, daß sie

a) die folgende Zusammensetzung besitzen:

$$SiO_2 : (0,20 \pm 0,10) \left[Al_2O_3 + B_2O_3\right] : (0,15 \pm 0,1) \left[Na_2O + K_2O\right] : (0,15 \pm 0,1)\ R_2O$$

ausgedrückt in Molverhältnisssen von Oxiden, wobei R gleich Tetramethylammonium ist,

und

b) im Röntgenbeugungsdiagramm die in Tabelle 1 aufgeführten charakteristischen Signale aufweisen:

Tabelle 1

| Netzebenenabstände d [Å] | relative Intensität $I/I_o$ |
|---|---|
| 11,50 ± 0,2 | sehr stark |
| 7,56 ± 0,1 | mittel |
| 6,62 ± 0,1 | stark |
| 6,32 ± 0,1 | schwach |
| 5,74 ± 0,1 | schwach |
| 4,56 ± 0,1 | mittel |
| 4,33 ± 0,1 | stark |
| 3,76 ± 0,1 | sehr stark |
| 3,58 ± 0,1 | stark |
| 3,31 ± 0,1 | schwach |
| 3,15 ± 0,1 | mittel |
| 2,84 ± 0,1 | sehr stark |
| 2,68 ± 0,1 | schwach |
| 2,48 ± 0,1 | schwach |

wobei $I_o$ die Intensität des stärksten Signals ist.

2. Boro-Alumosilicate nach Anspruch 1, dadurch gekennzeichnet, daß das Aluminium-Bor-Verhältnis

$$\frac{Al_2O_3}{Al_2O_3 + B_2O_3} = 0.4 - 0.99$$ ist, ausgedrückt in Molverhältnissen der Oxide.

3. Boro-Alumosilicate nach Anspruch 1 oder 2, dadurch gekennzeichnet, daß das Aluminium-Bor-Verhältnis

$$\frac{Al_2O_3}{Al_2O_3 + B_2O_3} = 0.75 - 0.99$$ ist, ausgedrückt in Molverhältnissen der Oxide.

4. Verfahren zur Herstellung von Boro-Alumosilicaten nach einem der Ansprüche 1 bis 3, dadurch gekennzeichnet, daß man eine Mischung aus Silicium-, Aluminium-, Bor-, Natrium-, Kalium- und Tetramethylammoniumverbindungen sowie Wasser herstellt, die folgende Zusammensetzung hat, ausgedrückt in Molverhältnissen der Oxide:

$SiO_2$ : (0,04 $\pm$ 0,025) $Al_2O_3$ : (0,04 $\pm$ 0,025) $B_2O_3$ : (0,2 $\pm$ 0,08) $Na_2O$ : (0,2 $\pm$ 0,15) $K_2O$ : (0,1 $\pm$ 0,08) $R_2O$: (25 $\pm$ 10) $H_2O$

wobei R gleich Tetramethylammonium ist, und diese Mischung in einem geschlossenen Gefäß erhitzt.

5. Verfahren nach Anspruch 4, dadurch gekennzeichnet, daß die zu erhitzende Mischung folgende Zusammensetzung hat, ausgedrückt in Molverhältnissen der Oxide:

$SiO_2$ : (0,035 $\pm$ 0,01) $Al_2O_3$ : (0,05 $\pm$ 0,015) $B_2O_3$: (0,2 $\pm$ 0,08) $Na_2O$ : (0,2 $\pm$ 0,15) $K_2O$ : (0,1 $\pm$ 0,08) $R_2O$: (25 $\pm$ 10) $H_2O$

wobei R gleich Tetramethylammonium ist.

6. Verwendung von Boro-Alumosilikaten nach einem der Ansprüche 1 bis 3 als Katalysatoren bei der Herstellung von $C_2$- bis $C_4$-Olefinen aus Methanol.